# FASCICULE DE BREVET EUROPEEN

(11) **EP 0 441 668 B1**
(45) Date de publication et mention de la délivrance du brevet: **26.07.1995**
(21) Numéro de dépôt: 91400090.6
(22) Date de dépôt: 16.01.1991
(51) Int. Cl.: A61B 17/60

(54) **Sabot de prise sacrée pour dispositif d'ostéosynthèse rachidienne**
Kreuzbeinstützblock für eine Vorrichtung zur Osteosynthese von Rückenwirbeln
Sacrum bearing block for rachidian osteosynthesis

(30) Priorité: 08.02.1990 FR 9001474
(43) Date de publication de la demande: 14.08.1991
(73) Titulaire: SOCIETE DE FABRICATION DE MATERIEL ORTHOPEDIQUE SOFAMOR, 75016 Paris (FR)
(72) Inventeur: Chopin, Daniel, F-62520 Le Touquet (FR)
(74) Mandataire: Martin, Jean-Paul

(56) Documents cités:
- EP-A- 0 348 272
- GB-A- 2 178 323
- US-A- 3 741 205

## Description

La présente invention a pour objet un sabot de prise sacrée pour dispositif d'osthéosynthèse rachidienne.

On sait que l'obtention d'une prise sacrée solide lors de l'ostéosynthèse rachidienne, reste un problème incomplètement résolu à ce jour. En effet dans sa plus grande partie l'os du sacrum est spongieux et porotique, surtout chez des sujets agés, et présente (voir GB-A-2 178 323) donc une faible résistance. Jusqu'à présent on a utilisé une vis unique positionnée en S1 (première vertèbre du sacrum), dans laquelle est insérée l'extrémité inférieure de la tige du dispositif correspondant, les autres vis étant enfoncées dans les deux vertèbres lombaires adjacentes au sacrum. Mais la faible résistance du sacrum, et le bras de levier important qui s'exerce sur la vis, développent des contraintes génératrices de risques d'arrachement de la vis.

Lorsqu'un tel arrachement se produit, il devient nécessaire que le chirurgien procède à une seconde intervention, avec généralement pose d'une seconde vis en S2 (seconde vertèbre du sacrum). Le dispositif devient encombrant. De plus cette seconde intervention chirurgicale peut entraîner des risques neurologiques en raison de la présence de nerfs en S2, est plus importante que la précédente et psychologiquement mal acceptée par le patient.

L'invention a donc pour but de proposer une solution satisfaisante à ce problème, assurant une prise sacrée suffisamment solide et sûre de manière à éviter la nécessité d'une seconde intervention.

Suivant l'invention, le sabot de prise sacrée comprend :
- un oeillet de réception d'une première vis, présentant un trou cylindrique ayant un axe d'inclinaison telle que lorsque le sabot est posé sur le sacrum, la vis s'insère dans le plateau sacré et vers l'axe supérieur de celui-ci,
- un corps en U délimitant un canal de réception d'une tige dudit dispositif, et un moyen de blocage de la tige dans ce corps en U,
- au moins un trou de passage d'une seconde vis dont l'axe est divergent par rapport à l'axe du trou de l'oeillet, de manière à être orienté vers un massif iliaque lorsque le sabot est posé sur le sacrum.

Ainsi la première vis s'enfonce dans une région du sacrum plus solide que celle qui recevait les vis utilisées jusqu'à présent, grâce à son inclinaison appropriée. Il en est de même pour la seconde vis qui diverge de la première et vient s'ancrer dans le massif iliaque.

L'insertion dans des directions divergentes des vis et dans les zones les plus solides du sacrum améliore donc considérablement l'ancrage, ce qui élimine normalement tout risque d'arrachement des vis et du sabot.

Suivant un premier mode de réalisation de l'invention, le trou de passage de la seconde vis est ménagé dans le fond du corps en U.

Suivant un second mode de réalisation, le trou de passage de la seconde vis aménagé dans une excroissance latérale au corps en U, et l'axe de ce trou est incliné d'un angle approprié sur un plan médian du canal dudit corps, passant par le fond de ce dernier, l'oeillet étant agencé sensiblement dans le prolongement du fond du canal.

L'inclinaison de l'axe du trou de l'oeillet sur le plan médian est de préférence de l'ordre de 20 à 30 degrés environ.

Le sabot selon l'invention et ses vis sont utilisables pour toutes les fixations sacrées, instrumentations lombo-sacrées, scolioses lombaires de l'adulte nécessitant une fixation jusqu'au sacrum, scolioses paralytiques, spondylolisthésis.

D'autres caractéristiques et avantages de l'invention apparaîtront au cours de la description qui va suivre, faite en référence aux dessins annexés qui en illustrent plusieurs modes de réalisation à titre d'exemples non limitatifs.

La figure 1 est une vue en perspective d'une première forme de réalisation du sabot selon l'invention, sans ses vis.

Les figures 2 à 4 sont des vues en perspectives similaires à la Fig. 1 de trois autres modes de réalisation du sabot selon l'invention sans leurs vis.

La figure 5 est une vue en perspective d'une cinquième forme de réalisation du sabot selon l'invention, équipée de deux vis.

La figure 6 est une vue en élévation d'un sacrum, du côté de la première vertèbre lombaire, ce sacrum étant muni de deux sabots conformes à la réalisation de la Fig.5 et équipés de leurs vis.

La figure 7 est une vue en élévation d'un dispositif d'ostéosynthèse rachidienne comprenant deux sabots conformes à la Fig. 1 et mis en place sur le sacrum ainsi que sur les deux vertèbres lombaires adjacentes.

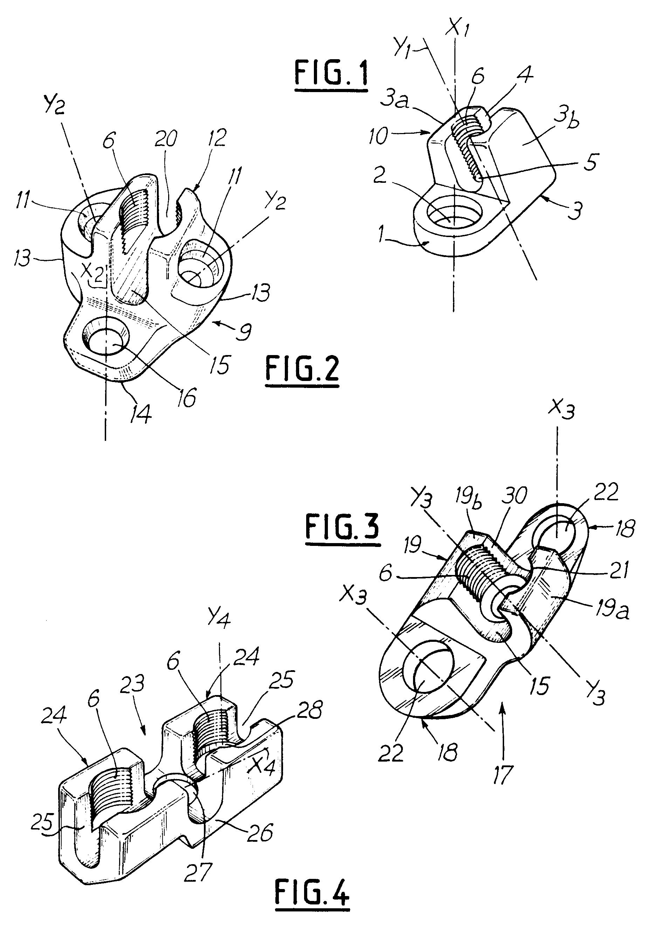

Le sabot 10 représenté à la Fig. 1 est destiné à une prise sacrée dans un dispositif d'ostéosynthèse rachidienne. Il comprend un oeillet 1 de réception d'une première vis non représentée, dans lequel est ménagé un trou cylindrique 2 ayant un axe X1 d'inclinaison telle que lorsque le sabot 10 est posé sur le sacrum, la vis correspondante, traversant le trou 2, s'insère dans le plateau sacré et vers l'axe supérieur de celui-ci;
- un corps 3 en U, réalisé monopièce avec l'oeillet 1, délimitant un canal 4 de réception d'une tige non représentée du dispositif,
- un trou 5 de passage d'une seconde vis non représentée, ménagé dans le fond du corps 3, et dont l'axe Y1 est divergent par rapport à l'axe X1, de manière à être orienté vers le massif iliaque lorsque le sabot est posé sur le sacrum.

L'angle entre les deux axes X1 et Y1 peut largement varier.

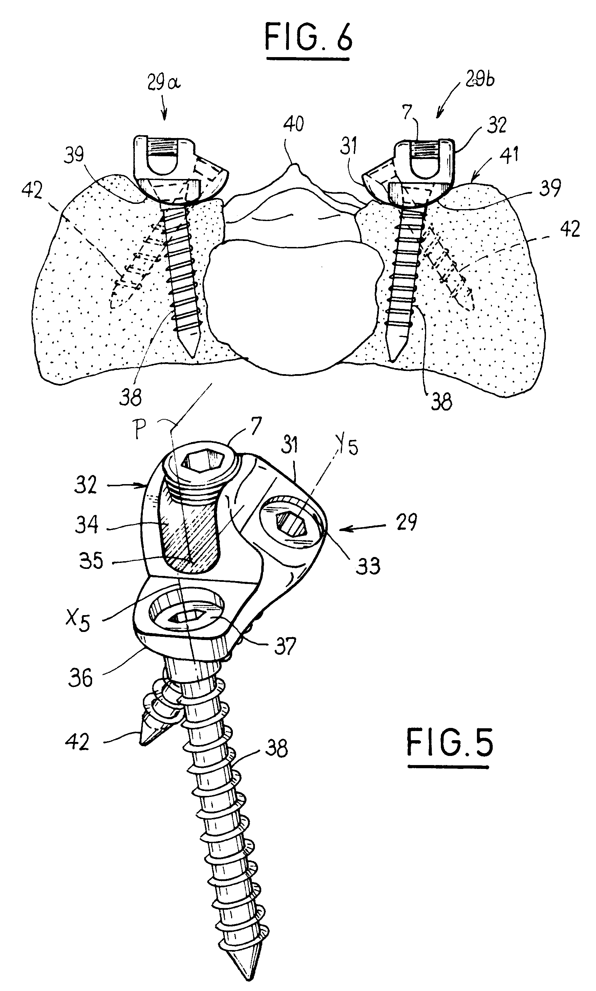

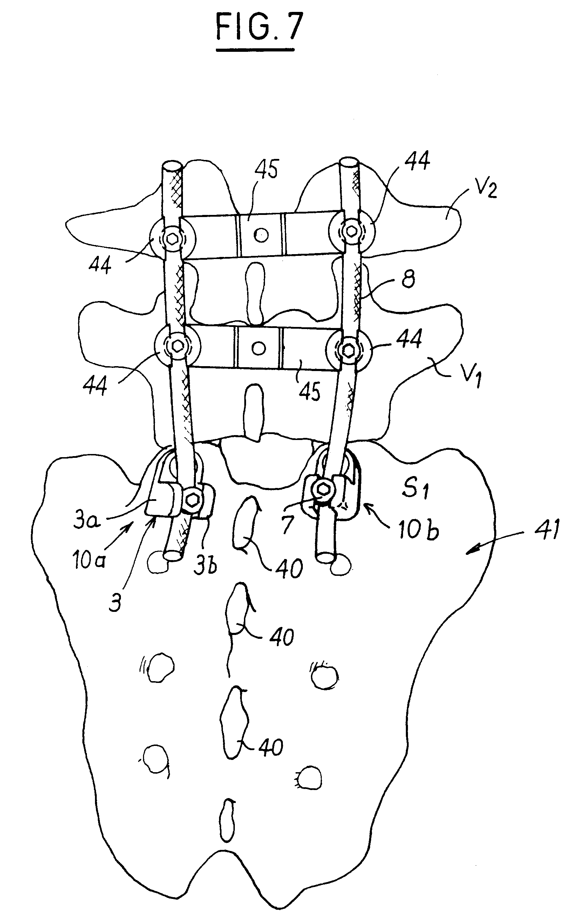

Sur les parois intérieures du canal 4 sont formées des sections taraudées 6 aptes à recevoir un bouchon fileté correspondant tel que 7 (Fig.5 et 6) de blocage d'une tige 8 (Fig. 7) insérée dans le canal 4.

Dans le second mode de réalisation du sabot 9, illustré à la Fig. 2, celui-ci comprend deux trous 11 de passage de la seconde vis et d'une troisième vis, ménagés symétriquement par rapport au corps central 12 en U, et à son canal 20, dans des excroissances latérales 13. L'oeillet 14 est agencé latéralement au corps 13 et sensiblement dans le prolongement de son fond 15. L'axe X2 du trou 16 de l'oeillet 14 est orienté de manière que lorsque le sabot 9 est posé sur le sacrum, la vis correspondante s'insère dans le plateau sacré et vers l'axe supérieur de celui-ci, comme dans le cas du sabot 10. Les axes Y2 des trous 11 sont de leur côtés inclinés d'un angle approprié sur un plan médian du canal 4 passant par son fond 15 et les vis correspondantes convergent l'une vers l'autre, l'une d'elles s'enfonçant dans le massif iliaque lorsque le sabot 9 est posé.

La Fig. 3 montre une troisième forme de réalisation du sabot 17, qui comprend deux oeillets 18 disposés de part et d'autre du corps 19 en U et sensiblement dans le prolongement du fond 15 du canal 30, avec toutefois un décalage latéral par rapport à l'axe du fond 15. Les parois 19a, 19b, du corps 19 présentent une inclinaison déterminée sur le plan général des deux oeillets 18, soit d'un côté, soit de l'autre. Cette inclinaison est de préférence égale, pour des raisons de commodité de montage, à celle de l'axe Y3 d'un trou 21 percé dans le fond 15 du canal 4 et destiné à recevoir une vis non représentée. Les axes X3 ont des inclinaisons différentes sur le plan général des oeillets 18, de sorte que les trois vis insérées dans les trous 21 et 22 forment un ensemble divergent.

Dans la troisième forme de réalisation représentée à la Fig. 4, le sabot 23 comporte deux corps 24 en U dont les canaux 25 sont alignés et qui sont reliés par un oeillet 26 dans lequel est ménagé un trou 27 de vis. L'axe X4 du trou 27 présente une inclinaison similaire à celle des axes X3, X2, X1 de façon que la vis correspondante puisse s'insérer également dans le plateau sacré. Un trou 28 de passage d'une seconde vis est ménagé dans le fond de l'un des corps 24, et son axe Y4 est incliné de manière que la vis correspondante puisse s'insérer dans le massif iliaque lorsque le sabot 23 est posé sur le sacrum.

Dans ce mode de réalisation comme dans les trois précédents, des taraudages 6 sont ménagés sur les parois intérieures en vis-à-vis des corps en U 24, et sont destinés à recevoir un bouchon fileté tel que 7 de blocage de la tige 8 dans les corps 24.

La Fig.5 illustre un cinquième mode de réalisation de l'invention, dans lequel le sabot 29 comprend une excroissance 31 latérale aux corps 32 en U. Un trou 33 est ménagé dans l'excroissance 31 avec son axe Y5 incliné d'un angle approprié sur un plan médian P du canal 34 du corps 32, passant par le fond 35 de ce dernier. Cette inclinaison de l'axe X5 sur le plan P peut être de préférence de l'ordre de 20 à 30 degrés environ. Le sabot 29 est complété par un oeillet 36 dont le trou 37 a un axe X5 orienté de manière similaire aux axes X4, X3,..., de manière que la vis correspondante 38 puisse venir s'insérer dans le plateau sacré et vers l'axe supérieur de ce dernier (Fig.6). Comme dans les réalisations précédentes, les parois du corps 32 sont munies de sections taraudées pour recevoir le bouchon fileté 7 de blocage de la tige 8 (Fig.7), et le sabot 29 est monopiéce.

La surface 39 du corps 32 et de l'excroissance 31, destinée à prendre appui sur le sacrum 41, est avantageusement hémisphérique, pour s'adapter à l'anatomie du sacrum 31.

Le trou 33 peut recevoir une vis 42 venant s'insérer dans le massif iliaque, comme visible à la Fig.6.

La Fig.7 montre un dispositif d'ostéosynthèse rachidienne complet avec prise sacrée de deux sabots 10 en S1, ainsi que de vis 44 sur les deux premières vertèbres lombaires V1, V2. Les deux parois 3a, 3b du corps 3 ont sur le plan général de l'oeillet 1, une inclinaison d'un côté ou de l'autre selon l'emplacement prévu pour le sabot sur le sacrum, vis-à-vis de la ligne des épineuses 42. Sur la Fig. 7 le sabot de gauche 10a correspond à celui de la Fig. 1, les parois 3a, 3b et l'axe Y1 du trou 5 ayant une inclinaison du côté des épineuses 42 (inclinaison dite "en dedans" par le chirurgien). Symétriquement, le second sabot 10b disposé sur la droite de la Fig. 7, a ses parois inclinées vers les épineuses 42.

Un sabot tel que 10 n'est donc pas utilisable indifféremment d'un côté ou de l'autre des épineuses 42, un jeu de chaque sabot 10a, 10b étant nécessaire.

Il en est de même pour les sabots 17 et 29, en raison de l'inclinaison des parois 19a, 19b pour le sabot 17, et de la position latérale de l'excroissance 31 pour le sabot 29. On voit en effet sur la Fig.6 un sabot 29a identique au sabot 29 de la Fig.5, dont l'oeillet 36 est placé du côté de la première vertèbre lombaire V1 et son excroissance 31 positionnée du côté des épineuses 40. Par contre, le second sabot 29b placé de l'autre côté des épineuses 42, a son excroissance 31 placée de l'autre côté du corps 32 par rapport à l'excroissance 31 des sabots 29 et 29a, et du côté des épineuses 40. De ce fait, les inclinaisons des axes des trous 33 et des vis correspondantes 42 sont opposées, ce qui leur permet de s'enfoncer dans les parties visées des ailerons sacrés.

On notera qu'en revanche les sabots 9 et 23 sont symétriques par rapport à un plan médian de leur corps en U, et peuvent donc être utilisés indifféremment d'un côté ou de l'autre de la ligne des épineuses 40.

La mise en place d'un sabot selon l'invention s'effectue de la manière suivante.

Le trajet de la première vis telle que 38, traversant l'oeillet, est préparé. Il s'étend de la partie externe du pied de l'articulaire de S1 et se dirige obliquement vers la ligne des épineuses 40, avec une inclinaison par exemple de 10 à 15 degrés légérement ascendante. Ainsi la vis 38 peut se diriger au travers du pédicule de S1 vers le plateau supérieur de S1 en os sous chondral, de façon à réaliser une prise particulièrement solide. Le sabot est alors fixé en plaçant cette première vis 38 par l'oeillet correspondant tel que par exemple 36, le corps en U tel que 3 ayant ses parois inclinées du côté des épineuses 40, comme visible notamment à la Fig.7.

Le second orifice est ensuite préparé en plaçant le sabot parallèlement à la ligne médiane des épineuses 40, ou obliquement de 5 à 10 degrés vers l'extérieur. La direction du deuxième orifice est donnée par l'inclinaison du canal tel que 4 (Fig.1 et 3) du sabot. Durant cette préparation, le sabot est tenu par un instrument adapté, connu en soi, et la deuxième vis telle que 42 est alors mise en place. Elle se dirige vers le massif iliaque, et doit être suffisamment longue pour s'approcher de la corticale de l'aileron sans la franchir. Sur les sujets jeunes, avec un os de bonne qualité, cette prise est suffisante.

Il reste au chirurgien à introduire chaque tige courbée 8 dans le corps ouvert (3, 12, ...), ce qui se fait aisément sans risquer de déplacer la prise du sabot. Le blocage de chaque tige 8 est ensuite assuré par la mise en place des bouchons filetés 7.

Le dispositif de fixation visible à la Fig.7 comprend, de façon connue en soi, les vis 44 enfoncées dans les pédicules des vertèbres V1 et V2, ainsi que des barettes 45 de liaison transversale. Ces éléments ne font pas partie de la présente invention et ne nécessitent donc pas de description détaillée.

Le bouchon fileté 7 et les taraudages 6 sont avantageusement conformes à la demande de brevet FR-A-2 633 177 déposée par la Demanderesse. Ces moyens de blocage peuvent être remplacés par tout autre système équivalent, sans sortir du cadre de la présente invention.

Chez les adolescents et les adultes jeunes, la prise sacrée décrite ci-dessus est généralement suffisante. En revanche, chez des patients plus âgés, lorsque le sacrum est porotique, cette prise de la tige 8 doit être renforcée. On utilise alors une vis iliaque supplémentaire, en combinaison avec le sabot selon l'invention. La tige 8 est donc fixée à la fois par le sabot et par la vis iliaque. Toutefois la mise en oeuvre de cette vis iliaque ne fait pas partie de la présente invention et ne sera donc pas davantage décrite.

Parmi diverses variantes possibles, il convient de signaler que le sabot peut présenter un trou de passage supplémentaire pour une troisième vis, dont l'axe est divergent par rapport à l'axe du trou de l'oeillet, de manière que la troisième vis correspondante soit orientée comme la seconde, dans le massif iliaque lorsque le sabot est posé sur le sacrum. Dans le sabot 23 (Fig.4) cette troisième vis traverserait ainsi un trou ménagé dans le fond du deuxième corps 24 en U.

## Revendications

1. Sabot (10, 9,...) de prise sacrée pour dispositif d'ostéosynthèse rachidienne, comprenant :
- un oeillet (1, 14,...) de réception d'une première vis (38), présentant un trou cylindrique (2, 16,...),
- un corps (3, 12,...) en U délimitant un canal (4, 20,...) de réception d'une tige dudit dispositif, et un moyen (7) de blocage de la tige (8) dans ce corps en U, caractérisé en ce qu'il comprend :
- au moins un trou (5, 11,...) de passage d'une seconde vis (42) dont l'axe (Y1, Y2,...) est divergent par rapport à l'axe (X1, X2,...) du trou de l'oeillet, de manière à être orienté vers un massif iliaque lorsque le sabot (10, 9,...) est posé sur le sacrum, ledit trou cylindrique (2, 16,...) ayant un axe (X1, X2,...) d'inclinaison telle que lorsque le sabot (10, 9,...) est posé sur le sacrum (41), la vis (38) s'insère dans le plateau sacré et vers l'axe supérieur de celui-ci.

2. Sabot selon la revendication 1, caractérisé en ce que le trou (5) de passage de ladite seconde vis (42) est ménagé dans le fond du corps (3) en U.

3. Sabot selon la revendication 1, caractérisé en ce qu'il comporte deux corps (24) en U alignés pour l'insertion de la tige (8), dans l'un au moins desquels est formé un trou (28) de passage de ladite seconde vis (42), et entre lesquels est agencé l'oeillet (26).

4. Sabot selon la revendication 1, caractérisé en ce qu'il comprend deux trous (11) de passage de la seconde et d'une troisième vis, symétriques par rapport au corps (12) en U, et l'oeillet (14) est agencé latéralement à ce dernier et sensiblement dans le prolongement de son fond (15).

5. Sabot (29) selon la revendication 1, caractérisé en ce que le trou (33) de passage de ladite seconde vis (42) est ménagé dans une excroissance (31) latérale au corps (32) en U, et l'axe (Y5) de ce trou est incliné d'un angle approprié sur un plan médian (P) du canal (34) dudit corps, passant par le fond (35) de ce dernier, l'oeillet (36) étant agençé sensiblement dans le prolongement du fond (35) du canal (34).

6. Sabot selon la revendication 5, caractérisé en ce que l'inclinaison de l'axe (X5) du trou (37) de l'oeillet (36) sur ledit plan médian (P) est de l'ordre de 20 à 30 degrés environ.

7. Sabot selon l'une des revendications 5 et 6, caractérisé en ce que la surface (39) du corps (32) en U et de l'excroissance (31) destinée à prendre appui sur le sacrum (41) est hémisphérique pour s'adapter à l'anatomie de ce dernier.

8. Sabot selon la revendication 1, caractérisé en ce qu'il comprend deux oeillets (18) disposés de part et d'autre du corps (19) en U et sensiblement dans le prolongement du canal (30) de ce dernier ou décalés par rapport à ce canal.

9. Sabot selon l'une quelconque des revendications 1, 2 et 8, caractérisé en ce que les parois (3a, 3b ; 19a, 19b) délimitant le corps (3; 19) en U sont inclinées, d'un côté ou de l'autre, sur le plan général de l'oeillet (1; 18).

10. Sabot selon l'une quelconque des revendications 1 à 9, caractérisé en ce que ledit moyen de blocage est un bouchon fileté (7) pouvant être vissé dans un taraudage correspondant (6) des parois du corps en U.

11. Ensemble constitué par un sabot (10, 9, 17, 23, 29) selon l'une quelconque des revendications 1 à 10 et par lesdites premières (38) et seconde vis (42) et le cas échéant une troisième vis, traversant le fond d'un second corps en U du sabot.

## Claims

1. Sacral support saddle (10, 9, etc.) for a spinal osteosynthesis device, comprising:
- an eye (1, 14, etc.) for receiving a first screw (38), and having a cylindrical hole (2, 16, etc.)
- a U-shaped body (3, 12, etc.) delimiting a channel (4, 20, etc.) for receiving a rod of the said device, and a means (7) for locking the rod (8) in this U-shaped body, characterized in that it comprises
- at least one hole (5, 11, etc.) for passage of a second screw (42), whose axis (Y1, Y2, etc.) is divergent relative to the axis (X1, X2, etc.) of the hole of the eye, in such a way as to be oriented towards an iliac mass when the saddle (10, 9, etc.) is positioned on the sacrum the said cylindrical hole (2, 16, etc.) having an axis (X1, X2, etc.) of inclination such that, when the saddle (10, 9, etc.) is positioned on the sacrum (41), the screw (38) is inserted into the sacral plate and towards the upper axis of the latter.

2. Saddle according to Claim 1, characterised in that the hole (5) for passage of the said second screw (42) is made in the base of the U-shaped body (3).

3. Saddle according to Claim 1, characterised in that it comprises two U-shaped bodies (24) aligned for the insertion of the rod (8), in at least one of which there is a hole (28) for passage of the said second screw (42), and between which the eye (26) is arranged.

4. Saddle according to Claim 1, characterised in that it comprises two holes (11) for passage of the second and of a third screw, which holes are symmetrical relative to the U-shaped body (12), and the eye (14) is arranged on the side of the latter and essentially in the extension of its base (15).

5. Saddle (29) according to Claim 1, characterised in that the hole (33) for passage of the said second screw (42) is made in a projection (31) on the side of the U-shaped body (32), and the axis (Y5) of this hole is inclined by a suitable angle to a median plane (P) of the channel (34) of the said body, passing through the base (35) of the latter, the eye (36) being arranged essentially in the extension of the base (35) of the channel (34).

6. Saddle according to Claim 5, characterised in that the inclination of the axis (X5) of the hole (37) of the eye (36) to the said median plane (P) is of the order of about 20 to 30 degrees.

7. Saddle according to one of Claims 5 and 6, characterised in that that surface (39) of the U-shaped body (32) and of the projection (31) which is intended to bear on the sacrum (41) is hemispherical so as to match the anatomy of the latter.

8. Saddle according to Claim 1, characterised in that it comprises two eyes (18) arranged on each side of the U-shaped body (19) and essentially in the extension of the channel (30) of the latter or offset relative to this channel.

9. Saddle according to any one of Claims 1, 2 and 8, characterised in that the walls (3a, 3b; 19a, 19b) delimiting the U-shaped body (3; 19) are inclined, on one side or the other, to the general plane of the eye (1; 18).

10. Saddle according to any one of Claims 1 to 9, characterised in that the said locking means is a threaded plug (7) which can be screwed into a corresponding tapping (6) in the walls of the U-shaped body.

11. Assembly consisting of a saddle (10, 9, 17, 23, 29) according to any one of Claims 1 to 10 and of the said first (38) and second (42) screws and, if appropriate, a third screw, passing through the base of a second U-shaped body of the saddle.

## Patentansprüche

1. Kreuzbeinstützblock (10, 9, ...) für eine Vorrichtung zur Osteosynthese von Rückenwirbeln, mit:
- einem Auge (1, 14, ...) zum Aufnehmen einer ersten Schraube (38), das ein zylindrisches Loch (2, 16, ...) zeigt,
- einem Körper (3, 12, ...) in U-Form, der einen Kanal (4, 20, ...) zum Aufnehmen einer Stange der Vorrichtung begrenzt, und einer Einrichtung (7) zum Blockieren der Stange (8) in dem U-Körper, dadurch gekennzeichnet, daß er aufweist:
- wenigstens ein Loch (5, 11, ...) zum Durchlaß einer zweiten Schraube (42), deren Achse (Y1, Y2, ...) in bezug auf die Achse (X1, X2, ...) der Öffnung des Auges abweicht, derart, daß sie gegen einen Hüftblock ausgerichtet ist, wenn der Stützblock (10, 9, ...) auf das Kreuzbein gelegt ist, wobei das zylindrische Loch (2, 16, ...) eine Neigungsachse (X1, X2, ...) derart hat, daß, wenn der Stutzblock (10, 9, ...) auf dem Kreuzbein (51) liegt, die Schraube (38) sich in die Kreuzplatte und in Richtung auf deren oberer Achse einfügt.

2. Stützblock nach Anspruch 1, dadurch gekennzeichnet, daß das Loch (5) zum Durchlassen der zweiten Schraube (42) im Boden des U-Körpers (3) angebracht ist.

3. Stützblock nach Anspruch 1, dadurch gekennzeichnet, daß er zwei Körper (24) in U-Form aufweist, die zum Einsetzen der Stange (8) ausgerichtet sind, wobei in wenigstens einem von ihnen ein Loch (28) zum Durchlassen der zweiten Schraube (42) gebildet ist, und wobei zwischen ihnen das Auge (26) vorgesehen ist.

4. Stützblock nach Anspruch 1, dadurch gekennzeichnet, daß er zwei Löcher (11) zum Durchlaß der zweiten und einer dritten Schraube aufweist, symmetrisch in bezug auf den U-Körper (12), und daß das Auge (14) seitlich an diesem und im wesentlichen in der Verlängerung seines Bodens (15) angebracht ist.

5. Stützblock (29) nach Anspruch 1, dadurch gekennzeichnet, daß das Loch (33) zum Durchlaß der zweiten Schraube (42) in einer seitlichen Ausstülpung (31) des U-Körpers (32) angebracht ist und daß die Achse (Y5) dieses Loches um einen geeigneten Winkel um eine Mittenebene (P) des Kanals (34) des Körpers geneigt ist, wobei sie durch den Boden (35) des letzteren läuft, wobei das Auge (36) im wesentlichen in der Verlängerung des Bodens (35) des Kanals (34) angeordnet ist.

6. Stützblock nach Anspruch 5, dadurch gekennzeichnet, daß die Neigung der Achse (X5) des Loches (37) des Auges (36) auf der Mittenebene (P) in der Größenordnung von ungefähr 20 bis 30 Grad liegt.

7. Stützblock nach einem der Ansprüche 5 und 6, dadurch gekennzeichnet, daß die Oberfläche (39) des U-Körpers (32) und der Ausstülpung (31), dazu bestimmt, an dem Kreuzbein (41) anzuliegen, halbkugelförmig sind, um sich an die Anatomie des letzteren anzupassen.

8. Stützblock nach Anspruch 1, dadurch gekennzeichnet, daß er zwei Augen (18) aufweist, die beidseits des U-Körpers (19) und im wesentlichen in der Verlängerung des Kanals (30) des letzteren angeordnet oder in bezug auf diesen Kanal versetzt sind.

9. Stützblock nach einem der Ansprüche 1, 2 und 8, dadurch gekennzeichnet, daß die Wände (3a, 3b; 19a, 19b), die den U-Körper (3; 19) begrenzen, geneigt sind, zur einen Seite oder zur anderen, auf der allgemeinen Ebene des Auges (1; 18).

10. Stützblock nach einem der Ansprüche 1 bis 9, dadurch gekennzeichnet, daß die Blockiereinrichtung ein Gewindestopfen (7) ist, der in ein entsprechendes Gewinde (6) der Wände des U-Körpers geschraubt werden kann.

11. Anordnung, gebildet durch einen Stützblock (10, 9, 17, 23, 29) gemäß einem der Ansprüche 1 bis 10 und durch die erste (38) und zweite Schraube (42) und gegebenenfalls eine dritte Schraube, die den Boden eines zweiten U-Körpers des Stützblocks durchqueren.
